# EUROPEAN PATENT SPECIFICATION

(11) **EP 2 716 172 B1**
(45) Date of publication and mention of the grant of the patent: **16.01.2019**
(21) Application number: 13187017.2
(22) Date of filing: 02.10.2013
(51) Int. Cl.: A41D 1/00, A41D 13/00, A41D 27/10

(54) **Item of clothing**
Kleidungsstück
Vêtement

(30) Priority: 02.10.2012 DE 102012218068
(43) Date of publication of application: 09.04.2014
(73) Proprietor: Adidas AG, 91074 Herzogenaurach (DE)
(72) Inventor: Watson, Trudy Anne, Portland, OR, 97215 (US); Yeomans, Deborah, 91074 Herzogenaurach (DE); O Mahony, David, 90419 Nürnberg (DE); Tarrier, James Michael, 90408 Nürnberg (DE); Seider, Bettina, 90766 Fürth (DE)
(74) Representative: Wegner, Hans

(56) References cited:
- US-A- 2 554 380
- US-A- 2 941 210
- US-A- 3 137 860
- US-A1- 2005 054 941
- US-A1- 2005 275 416

## Description

### 1. Technical field

The present invention relates to an item of clothing with a receptacle for a sensor as well as a method for the manufacture of an item of sports clothing.

### 2. Prior art

Items of clothing are increasingly being provided with sensors, particularly in the area of sports, but also in the stationary or ambulant monitoring of patients. These sensors are able to measure physiological data of a wearer of such an item of clothing. For example, the heart rate, electrocardiogram (ECG) signals or respiratory signals belong to this physiological data, but also the current state of motion and the body temperature.

For example, the heart rate is measured through two electrodes that make contact with the skin of a human. The human heartbeat, in particular its so-called RR-Interval, brings about voltage changes on the skin, which can be measured by the two electrodes.

The measurement of respiration takes place through meander-shaped electrical conductors which can be arranged in the chest and/or abdomen region and respectively represent an electrical coil and are connected to an electrical oscillator. Due to the respiratory motions the circumference of the chest and abdomen alter and thus the length of the conductors, the inductance of the coils and finally the oscillation frequency. The alteration of the oscillation frequency can be evaluated and permits conclusions to be made with regard to the respiratory motions.

The state of motion of a human can be detected by way of position sensors or acceleration sensors. Position sensors are able to provide data with respect to their position in space, while acceleration sensors measure acceleration acting upon them. The sensors can be arranged at individual body parts such as the limbs for instance, in order to measure the motion and/or position of the body parts. Distance sensors can be used in order to measure the distance between individual body parts in relation to one another.

It is desirable that the sensors affixed to the item of clothing remain fixed, i.e. do not shift. For example, a shifting of an above described electrode for heart rate measurement can lead to the contact to the skin being interrupted or lost entirely so that a measurement of the voltage on the skin generated by the heart beat is no longer possible.

It may also occur that a sensor distances itself too far away from the organ, the physiological signals of which are to be measured. For example, an above described object for measuring respiratory motion can shift too far upwards in the direction of the armpits or may tilt, so that a determination of the respiratory motion is no longer possible. In a different example, an electrode for measuring the heart rate may, upon wearing an item of clothing that is equipped with such electrodes, distance itself from the heart to such an extent that a measurement of the voltage on the skin generated by the heartbeat is no longer possible.

The desire for preferably fixed, i.e. non-shifting sensors upon wearing items of clothing that are provided with sensors is opposed by the fact that such items of clothing are worn precisely in situations in which body motions occur that precisely favor shifting. For example, the wearer of an item of clothing for a sport sometimes practices complex and strong movements. For example, a football player raises their arms above their head when performing a throw-in, so that the trunk portion of their jersey experiences a force in the upwardly direction, i.e. in the direction of the head. With a tennis player, a once-sided force occurs at the upper body clothing when hitting the ball, which can lead to a twisting of the upper body clothing in relation to the skin. Patients who wear items of clothing that are provided with sensors during the night for purposes of monitoring, may unconsciously change their lying position and move, whereby this can lead to the shifting of the sensors.

In connection with the problem of the shifting of the sensor or the excess distancing from the organ to be measured, it is particularly important that the sensor is optimally positioned. If immediately after putting on the item of clothing provided with the sensor, said sensor is already positioned such that a signal can just about be measured, then a shifting caused by movement can be tolerated even less than if the sensor is positioned optimally immediately after putting on said item of clothing. If the sensor is incorrectly positioned immediately after putting on said item of clothing, i.e. is too far away from an organ that is to be measured for instance, then the sensor is unable to measure any signals from the outset.

US 2,554,380 D1 relates to a garment, wherein the body and sleeve portions of the garment are made of knitted fabric, independently of one another, and suitable gussets or inserts are embodied in said garment between the body and sleeve portions thereof, when the sleeves are stitched to the body portion. Said inserts are readily stretchable in a direction to permit free unrestricted movement of the wearer's arms in up-and-down directions, as well as in all other directions.

US 2005/0054941 A1 relates to a physiological monitoring garment. The physiological monitoring garment includes first and second elastic fabric portions. An elongate stretchable textile data/power bus is disposed between the first and second elastic fabric portions. The elongate stretchable textile data/power bus includes a plurality of integral conductors, woven, knitted, or braided along the length thereof. One or more sensors are connected to the elongate stretchable textile data/power bus.

Thus the object underlying the present invention is to position and fix a sensor that is provided in an item of clothing as best as possible so that when the item of clothing is worn a shifting of the sensors is reduced or avoided and that the measuring signal picked up by the sensor is not interrupted. It is a further object of the present invention to provide a method for manufacturing an item of sports clothing.

### 3. Summary of the invention

According to a first aspect of the present invention, this problem is solved by an item of clothing, whereby the item of clothing comprises as first textile area comprising at least one sensor, whereby the first textile area comprises a pocket for receiving the sensor, a second textile area and a third textile area that is arranged at least partially between the first and second textile area, whereby the third textile area is provided such that it isolates relative motions between the first textile area and the second textile area that occur when the item of clothing is worn, so that the sensor area remains substantially fixed relative to an area of a subjacent organ of a wearer of the item of clothing.

"Substantially fixed" means that when worn, the sensor does not shift to such an extent, that a measuring is no longer possible. The area of the subjacent organ can be the area in which the sensor is able to measure a measuring signal. If the sensor is a heart rate electrode, which lies against the skin, then this area can have a diameter of several centimeters on the skin, 10 cm for example. If the organ is the skin, then preferably only a portion of the skin with respect to which the sensor is to remain substantially fixed.

Different than with known items of clothing that are provided with sensor, the item of clothing according to the invention provides a third textile area which substantially isolates the relative motions between a first and second textile area. The first textile area, which comprises a sensor, can hereby move almost independently from the second textile area. The provision and arrangement of an isolating third textile area between the first and the second textile area reduces or avoids the forces occurring between the first and second textile areas due to relative motions between the first and second textile areas, which area caused through body motions of a wearer of the item of clothing.

The inventors have particularly realized that a shift always occurs when the item of clothing does not move with the skin, i.e. when the item of clothing stretches differently to the underlying skin upon occurring tensile, thrust and shearing forces. The fundamentally new concept, of guaranteeing, by way of the third textile area and its isolating arrangement between the first and second textile area, that the first textile area, which comprises a sensor, substantially moves with the skin and thus delivers signals continuously, underlies the present invention. Due to the isolation from the second textile area the first textile area substantially experiences the same tensile, thrust and shearing forces as the underlying skin and deforms in the same way as it, so that a shifting of the sensor is reduced or avoided.

The item of clothing according to the invention permits a measurement of physiological data during natural motion sequences, without there being a constant "loss" of the measuring signal. It is particularly important for real-time measurements that the measuring signal is not constantly lost, since the fixture of the sensor cannot be constantly monitored and corrected. For example, an athlete of a sport equipped with an item of clothing according to the invention does not need to leave the playing field in order to correct the position of a sensor. Even in relatively "rough" sports such as rugby, the fixture of the sensor is guaranteed.

In principal there is a large plurality of possibilities for the design of the third textile area to realize the new concept in accordance with the invention.

According to a first preferred embodiment of the invention, a material processed within the third textile area is more easily stretchable than a material processed within the first textile area.

"More easily stretchable" means that a section of the third textile area experiences a greater length variation or stretching upon application of forces that typically occur when wearing an item of clothing, than an equally large section of the first textile area. This can be achieved through different laminations, knitting, weaving or webbing patterns, fibers, material thicknesses or densities that differ in the first and third textile areas.

In that the third textile area has a more easily stretchable material than the first textile area, the tensile force occurring between the first and second textile areas is reduced and a shifting of the sensor in the first textile area is reduced or avoided. Simultaneously, the material processed in the first textile area can be less easily stretchable, in order to fix the sensor.

For example, the third textile area can have a smaller elasticity module than the first textile area. The elasticity module of a material indicates how far the material stretches under tensile force. Generally the elasticity module is defined as a quotient of the tensile force and the material expansion, i.e. a smaller elasticity module means that less tensile force is require for the same material expansion. Preferably, the third textile area has an elasticity module of 1/3 to ½ of the elasticity module of the first textile module, i.e. it can stretch up to two or three times the length of the first textile area under the same textile force.

A further possibility of the large plurality of possibilities for the implementation of the third textile areas provides, in accordance with an alternative, preferred embodiment of the invention, that the third textile area comprises an uneven structure. The third textile area thus does not lie close to the skin everywhere. The third textile area can, for instance, have folds (pleats) that are at right angles to the arising tensile force, which unfold through a tensile force occurring between a first and second textile area, i.e. are pulled straight. As a result of the folds, the third textile area comprises a sufficient fabric reserve, in order to minimize tensile forces through elongation or stretching and to isolate the first and second textile area. A fine, unfolding, crimped surface structure of the third textile area is also conceivable.

A further possibility of the large plurality of possibilities for the implementation of the third textile area provides, in accordance with an alternative, preferred embodiment of the invention, that the third textile area is provided such that, when the item of clothing is worn, it is distanced further away from the skin of the wearer of the item of clothing than the first textile area. The first and the second textile area can for instance be close fittingly cut to the wearer's body, while the third textile area has a different cut. As a result of this measure it is guaranteed that the third textile area has a sufficient fabric reserve in order to minimize tensile forces occurring as a result of elongation or stretching and to isolate the first and second textile area. Simultaneously, the first textile area lies sufficiently close to the skin of the wearer, in order to secure the contact of the sensor with the skin for instance and the second textile are lies closely enough, in order to ensure an appropriate fit of the item of clothing.

A further possibility of the large plurality of possibilities for the implementation of the third textile area provides, in accordance with an alternative, preferred embodiment of the invention, that the third textile area has a substantially different mechanical preliminary tension than the first and / or the second textile area. The mechanical preliminary tensions thus differ such that upon the motion of the wearer of the item of clothing, a sensor arranged in the first textile area is able to measure signals without interruption.

For example the third textile area can have a lower mechanical preliminary tension than the first and second textile area. In this manner the third textile area can deform more easily, when tensile stresses occur between the first and the second textile area while the item of clothing is worn. Occurring tensile forces can thus be minimized through an elongation or stretching of the third textile area and the first and second area are isolated.

A low mechanical preliminary tension of the third textile area can be achieved e.g. in that when connecting, e.g. sewing the third textile area to the first and / or second textile area the first and / or second textile area is stretched by means of tensile stress. In this manner, the third textile area receives a lower mechanical preliminary tension than the first and / or second textile area.

The above described alternative, preferred embodiments show that there is a large plurality of possibilities for the implementation of the third textile area. All possibilities have in common that the provision of a third textile area and its isolating arrangement between the first and second textile area ensure that the first textile area, which comprises a sensor, moves with the skin. In this manner, a shifting of the sensor is reduced or avoided. For example, it is guaranteed that a heart rate electrode remains in contact with the area of the skin in which electrical surface voltages brought about by the heard can be measured.

Naturally, a combination of the different approaches explained by way of example as well as other approaches to isolate the first and second area through a correspondingly implemented third area is conceivable.

In a preferred embodiment of the invention, the third textile area has an elongated section substantially along the direction of greatest stretching when the item of clothing is worn. The forces occurring between the first and second textile area are hereby effectively isolated.

The sensor can for instance be a skin electrode for measuring electrical voltages on the skin, as are used for measuring heart rate or for an ECG. The sensor can also be meander-shaped electrical conductors for measuring respiration, which can be arranged as described above. Also, the sensor can be a magnetometer which is able to measure physiological magnetic fields.

Preferably the organ is the skin or the heart or the lung of a wearer of the item of clothing. The organ can also be a body region such as the thorax, the abdomen or a limb such as a leg or an arm. Other organs, whose physiological data can be measured by a sensor, are conceivable.

Preferably, the third textile area is arranged between the first and the second textile area such that the first and the second textile area do not abut. However, this is not strictly necessary. For example, the third textile area can be a circumferential insert in the substantial shape of a cylindrical surface. This insert can, for example, surround a body part such as the arm or the armpit when wearing the item of clothing. As a result of the fact that the first and the second area do not abut, both areas are entirely isolated by the third textile area and the risk of a shifting of the sensor is further reduced.

Preferably, the item of clothing is an item of clothing for the upper part of the body, whereby the first textile area is a trunk area and whereby the second textile area is sleeve area. The item of clothing can be a T-shirt for example, as is worn in many kinds of sports, e.g. football, tennis or running. As a result of the fact that the item of clothing comprises sleeves, which are isolated from the trunk area through the third textile area from a force point of view, it can be worn visibly as a single item of clothing. The wearer does not need to wear the item of clothing provided with a sensor as an additional sleeveless undershirt under the actual sports clothing- The invention opens up the possibility to provide a plethora of items of clothing for different sports, different seasons such as summer and winter or indoor sports and outdoor sports with sensors for the first time, without there being an excessive risk of the sensors shifting.
Even more preferably, the third textile area is a circumferential sleeve portion. For example, it can be a sleeve portion in the shape of a cylindrical surface, which is arranged in the proximity of the armpit. Thus, even in a long-sleeved item of clothing, such as a winter jersey, the sleeve is hereby optimally isolated from the trunk area of the item of clothing.
The first textile area comprises a pocket for receiving the sensor. On the one hand, the sensor is hereby detachable so that it is not damaged when washing the item of clothing. On the other hand, it is optimally held in the pocket and positioned above the organ to be measured.

In non-claimed alternatives, the sensor may be sewn, woven, knitted or glued into the first textile area. If it is a sensor that is able to survive the cleaning of the item of clothing without damage, then this type of affixation is a simple manipulation of the item of clothing, since the item of clothing can simply be worn, without the need for the wearer to worry about the fixation of the sensor. Also a sensor that is sewn, woven, knitted or glued in cannot shift within the first textile area. In this manner, the sensor cannot be positioned incorrectly.
Preferably, the third textile area is a fabric. Fabrics have the advantage that their elasticity, e.g. expressed by means of its elasticity module, can easily be set by means of the kind of weaving technique and the yarn used.
Preferably, the first and second textile areas have the same material. The item of clothing hereby receives a substantially uniform appearance. For example, in a long-sleeved T-shirt, the sleeve, except for the third textile area arranged in the proximity of the armpit, can be made of the same material as the trunk area.

In a preferred embodiment of the invention, the third textile area comprises two different elasticity modules in two directions that are different from one another and oriented such that the direction of the smaller elasticity module is substantially parallel to the direction in which the greatest mechanical tension occurs when wearing the item of clothing. In this orientation of the third textile area, the third textile area can most easily stretch upon the occurrence of tensile forces, in order to reduce or avoid a shifting of the sensor in the first textile area.

Preferably, the item of clothing is designed to arrange the sensor such that it is positioned on one side of the torso of a wearer of the item of clothing when the item of clothing is worn. In this positioning the heartbeat of the wearer of the item of clothing can be easily measured for instance.

Preferably, the sensor can be a heart rate electrode, which is arranged in a lateral chest area. Even more preferably, two heart rate electrodes are arranged on opposite later chest areas.

In another preferred embodiment of the invention, the item of clothing is designed to arrange the sensor circumferentially so that it at least partially runs around the torso of the wearer of the item of clothing when the item of clothing is worn. For example, it can be a meander-shaped electrical conductor, which is used to measure respiration as described above, in that the alteration of the chest and / or abdomen circumference is measured.

An exemplary method for manufacturing an item of sports clothing comprises the following steps: a. Obtaining the stretching pattern of the skin of the wearer of the item of sports clothing during at least one sport-specific motion of the wearer; and b. Manufacturing the item of sports clothing, whereby the item of sports clothing exhibits a substantially similar stretching pattern to the stretching pattern of the skin during the sport-specific motion of the wearer and is suitable to receive a sensor. The sport-specific motion of the wearer is a specific motion of a sport. For example, the wearer can perform a throw-in during football as a specific motion, or the hitting of the ball in tennis. In this manner, an item of clothing can be manufactured that is adapted to a specific sport, which guarantees a reduced shifting of a sensor provided in said item of sports clothing when performing the sport.
The stretching pattern of the item of sports clothing is so similar to the stretching pattern of the skin that I sensor provided in the item of sports clothing, such as a heart rate electrode, remains substantially fixed relative to an area of a subjacent organ of a wearer of the item of sports clothing during a motion of the wearer of the item of sports clothing. This means that the sensor does not shift during the specific motions of the wearer such that a measurement is constantly no longer possible. The exemplary method permits the manufacture of an item of sports clothing, which is suitable to receive a sensor and thus enables a measurement of physiological data of a wearer of the item of sports clothing in real-time during natural motion sequences, without there being a constant "loss" of the measuring signal. For example, an athlete of a sport equipped with an item of clothing according to the invention does not need to leave the playing field in order to correct the position of a sensor. Even in relatively "rough" sports such as rugby, the fixture of the sensor is guaranteed. An item of sports clothing manufactured in accordance with the exemplary method on the one hand permits a good measuring signal of a provided sensor in a state of rest of a wearer of the item of clothing. On the other hand, a constant "loss" of the signal during motions of the wearer, e.g. during sport, is reduced or avoided.

In a preferred example of the method, step b. is designed such that the stretching patter of the item of sports clothing is provided such that when wearing the item of sports clothing a sensor received by said item of sports clothing is substantially fixed in relation to an area of a subjacent organ of a wearer of the item of clothing.

It is further preferred that the organ is the skin, the heart, the lung, the thorax or the abdomen of a wearer of the item of sports clothing. An organ is understood to also mean a body part such as the thorax or the abdomen, but for example also a limb such as a leg or an arm.

It is further preferred that the method comprises the step: Attaching a mounting for a sensor to the item of sports clothing. A mount can for example be a pocket or a clip.

It is further preferred that the method comprises the step: Attaching a sensor to the item of sports clothing. For example the sensor can be sewn, woven, knitted or glued into the item of sports clothing. However, the sensor can also be placed in a pocket of the item of sports clothing or be fixed to the item of sporty clothing by means of a clip or similar.

In a preferred example of the method, the mounting and/or the sensor are arranged in an area of little stretching in comparison to other areas of the stretching pattern of the item of sports clothing. In this manner it can be even better avoided or reduced that the sensor shifts during the specific motions of the wearer and that measuring is no longer possible.

In a preferred example of the method, a textile area is incorporated in an area of great stretching in comparison to other areas of the stretching pattern of the item of sports clothing, which is easier to stretch than other textile areas. Mechanical tensions between different areas of the item of sports clothing are hereby reduced and in particular it is ensured that a sensor provided in the item of sports clothing moves with the skin. In a preferred example of the method, obtaining the stretching pattern takes place in step a. by means of an optical method. For example, reference points can be applied on the skin, the relative change in distance of which is optically recorded during specific motions, in order to determine the stretching pattern.

Preferably, step b. of the method further comprises: Forming at least two textile areas of the item of sports clothing with different stretching behaviors. For example, a textile area, of the item of sports clothing, which exhibits great elasticity, can be provided in an area of great stretching of the skin. In an area little stretching of the skin, a textile area can be provided, which exhibits a correspondingly low elasticity. In this manner, the stretching pattern of the item of sports clothing can be adapted to the stretching pattern of the skin.

### 4. Short description of the Figures

In the following detailed description, the currently preferred embodiments of the item of clothing according to the invention are described with reference to the following Figures:
**Fig. 1****:** A schematic representation of a first embodiment of the present invention, in which the item of clothing according to the invention is an item of clothing for the upper part of the body;
**Fig. 2****:** A schematic representation of a motion of the item of clothing as in the embodiment of Fig. 1;
**Fig. 3****:** A schematic representation of an alternative embodiment of the item of clothing according to the invention with a fold-like structure;
**Fig. 4****:** A schematic representation of a further alternative embodiment of the item of clothing according to the invention comprising a third textile area that is distanced further away from the skin of a wearer of the item of clothing;
**Fig. 5****:** A schematic representation of a further embodiment of the item of clothing according to the invention comprising first and second textile areas that touch each other;
**Fig. 6****:** A schematic representation of a further embodiment of the item of clothing according to the invention comprising two different elasticity modules;
**Fig. 7****:** A representation of a further embodiment of the item of clothing according to the invention, in which the item of clothing is designed to arrange the sensor around the thorax of a wearer circumferentially;
**Fig. 8****:** A representation of a further embodiment of the item of clothing according to the invention, in which the item of clothing comprises a circumferential third textile area and an electrical conductor that is connected to the sensor;
**Fig. 9****:** An embodiment of the item of clothing according to the invention with a third textile area comprising two sections and a measurement data processing device;
**Fig. 10****:** A detailed representation of a section of a third textile area according to the embodiment of Fig. 9;
**Fig. 11****:** An illustration of a partial aspect of the performance of the exemplary method;
**Fig. 12****:** An illustration of a partial aspect of the performance of the exemplary method;
**Fig. 13****:** A detailed representation of a section of a third textile area in accordance with an embodiment;
**Fig. 14****:** A detailed representation of a section of a third textile area with a slot in accordance with an embodiment;
**Fig. 15****:** A partial view of an alternative embodiment of an item of clothing according to the invention;
**Fig. 16****:** A partial view of an alternative embodiment of an item of clothing according to the invention;
**Fig. 17****:** A representation with regard to locating an area of optimal position of a sensor;
**Fig. 18****:** An illustration of an aspect of locating an area of optimal position of a sensor;
**Fig. 19****:** An illustration of an aspect of locating an area of optimal position of a sensor for two different types of sport;

### 5. Detailed description of the preferred embodiments

In the following, the currently preferred embodiments of the present invention are described with reference to an item of clothing as well as a method.

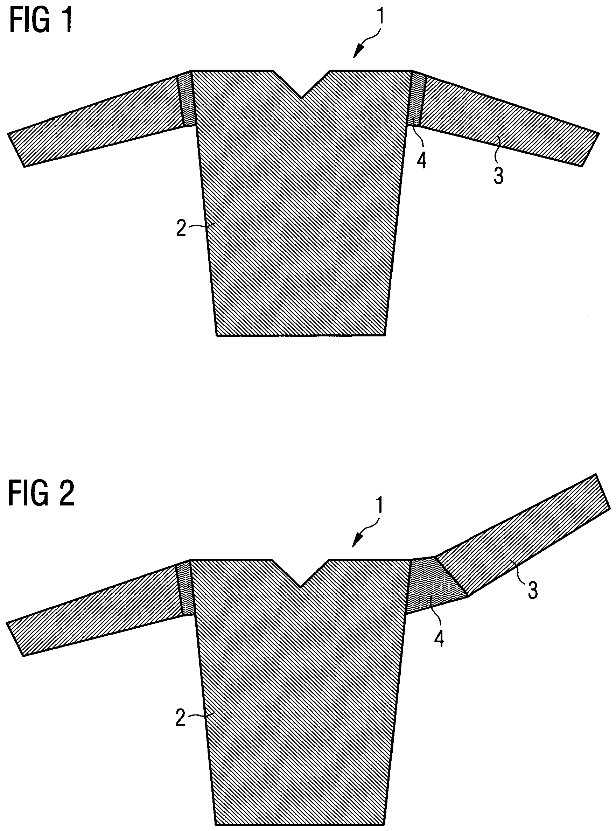

**Fig. 1** shows a schematic representation of an item of clothing **1** according to a first aspect of the present invention. In the embodiment of **Fig. 1** the item of clothing **1** is shown as a long-sleeved item of clothing for the upper part of the body, for example a football jersey for winter. Principally, the item of clothing can also be a short-sleeved shirt, for example a summer jersey, a running shirt, an undershirt, a vest, trousers, thus any item of clothing. Preferably, the item of clothing is functional clothing for sporting activities such as football or basketball.
The item of clothing is generally manufactured out of a textile fabric. The fabric can be a woven fabric, an interlaced yarn, a knitted fabric, a fleece, hence any kind of fabric manufactured out of textile fibers.
The item of clothing **1** shown in **Fig. 1** has a first textile area **2**, which comprises at least one sensor (not shown in **Fig. 1**). The first textile area **2** is manufactured from a textile fabric, as described above. In the item of clothing **1** in **Fig. 1** the first textile area is the trunk area of the item of clothing for the upper part of the body shown. In a different item of clothing according to the invention, for example a pair of trousers, the first textile area could be the thigh area for example.
The first textile area comprises at least one sensor (not shown in **Fig. 1**). The sensor is received through a mount (not shown in **Fig. 1**) in the form of a pocket. If the sensor requires contact with the skin, then the mounting can be arranged on the inside, i.e. the side facing the body of the item of clothing that is worn the correct way around. In such a case the mount permits the contact of the sensor to the skin, for example through one or more holes.
The first textile area **2** can also receive the sensor permanently, i.e. in a non-removable manner, but this does not form part of the claimed invention. For example, the sensor can be sewn, woven, knitted or glued into the first textile area.
The item of clothing **1** shown in **Fig. 1** further comprises a second textile area. The second textile area **3** is also manufactured out of a textile fabric, as described above. This can be the same material as that of the first textile area **2**. The second textile area **3** in **Fig. 1** is a sleeve-area of the item of clothing for the upper part of the body of **Fig. 1**. In a different item of clothing according to the invention, for example a pair of trousers, the second textile area could be the lower leg area for example. Furthermore, the item of clothing **1** shown in **Fig. 1** comprises a third textile area **4**. Also this third textile area **4** is manufactured out of a textile fabric, as described above. Principally, this can be a different textile fabric as that of the first and second textile area. The third textile area **4** shown in **Fig. 1** has the shape of a circumferential sleeve portion, i.e. it runs once around the arm of the wearer of the item of clothing **1**. For example, the sleeve portion **4** can be a circumferential insert substantially in the shape of a cylindrical surface. For example, the seams of the trunk and the sleeves of the T-shirt can be shortened in order to create space for the third textile area, so that the sleeve has the same length as before after the third textile area is inserted.

The third textile area **4** is provided such that it isolates relative motions between the first and second textile area when the item of clothing is worn, so that a sensor in the first textile area (not shown in **Fig. 1**) remains substantially fixed in relation to an area of a subjacent organ of the wearer of the item of clothing.

For example, the sensor can remain substantially fixed to an area of the subjacent skin. This is particularly advantageous for heart rate electrodes, which require a transition resistance between the skin and the electrode that is as low as possible. Depending on the use, the sensor can in addition or alternatively remain substantially fixed to an internal organ, e.g. the heart or the lung.

If the organ is the skin, for instance, then it is sufficient if the sensor remains substantially fixed to an area of the skin, which is immediately associated to the sensor. For example, it can be an area of skin that can have a diameter of several centimeters, for example 10 centimeters, on which the sensor lies and in relation to which the sensor remains substantially fixed. For the sensor to be in a position to measure the heart beat it is irrelevant whether other areas of the skin, such as on the leg, move in relation to the sensor or not.

The function of the third textile area **4** is illustrated in **Fig. 2**, which schematically shows a motion of the item of clothing **1** from the embodiment out of **Fig. 1**. In **Fig. 2** the item of clothing **1** is shown after the left arm of a wearer (not shown in **Fig. 2**) is raised. As a result of the provision and the arrangement of the third textile area **4** between the first textile area **2** and the second textile area **3** the motion of the second textile area **3** is substantially isolated from the first textile area **2**. Since the first textile area receives the sensor (not shown in **Fig. 2**), the motion of the second area **3** is not passed on to the sensor. It remains substantially fixed to the subjacent skin of the wearer, i.e. the measurements it performs are not negatively influenced.

As shown in **Fig. 2****,** the relative motion between the second textile area **3** and the first textile area **2** leads to a clear elongation or stretching of the third textile area **4**. This elongation or stretching can be caused in that the third textile area is more easily stretchable than the first textile area.

For example, the first textile area can comprise a smaller elasticity module than the first textile area. It is known that the elasticity module of a material indicates how far the material stretches under a tensile force. Generally the elasticity module is defined as a quotient of the tensile force and the material expansion, i.e. a smaller elasticity module means that less tensile force is require for the same material expansion. In the embodiment of **Fig. 1** and **Fig. 2** this means that the third textile area **4** stretches considerably more than the first and the second textile area.

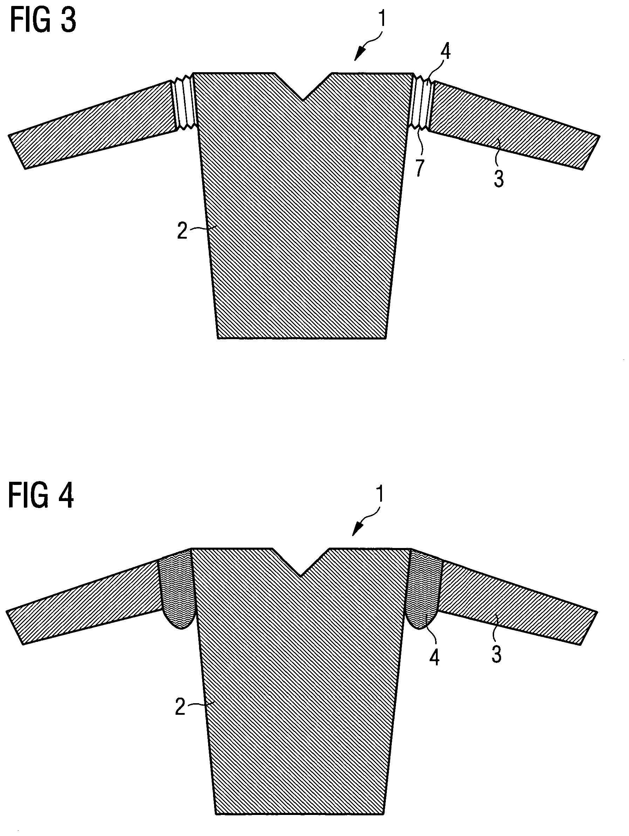

A further possibility of favoring an elongation or stretching of the third textile area **4** upon the occurrence of tensile forces is shown in **Fig. 3**. Here, the third textile area comprises a fold-like structure **7.** Such a structure could be a pleat, i.e. artificially created folds. Other structures are also conceivable, such as for example a crease fabric or a micro three dimensional structure. As shown in the embodiment of **Fig. 3**, these folds **7** can be arranged at right angles tot eh arising tensile force between the first and second textile area, circumferential around the sleeve for instance. In the event of a relative motion between the first and the second textile area, the folds **7** in the third textile area **4** and drawn-out, i.e. the third textile area is stretched. The folds 7 thus ensure that there is a sufficient fabric reserve of the third textile area **4** is provided for upon the occurrence of tensile forces, in order to effect an elongation or stretching.

A further possibility of favoring an elongation or stretching of the third textile area **4** upon the occurrence of tensile forces is shown in **Fig. 4**. Here the third textile area **4** is provided such that it is distanced further away from the skin of the wearer of the item of clothing than the first textile area when the item of clothing **1** is worn. In the embodiment of **Fig. 4** the third textile area **4** below the armpits is distanced further away from the skin of the wearer (not shown in **Fig. 4**) than the first and second textile area. In this embodiment the third textile area **4** is arranged partially between the first and second textile area. Partially, namely below the armpits, the third textile area is not arranged between the first and second textile area. As a result of this measure, the third textile area **4** has a sufficient fabric reserve in order to effect an elongation or stretching in the event of occurring tensile forces.

A further possibility of favoring an elongation or stretching of the third textile area **4** upon the occurrence of tensile forces lies in that the third textile area **4** has a different mechanical preliminary tension than the first and / or the second textile area. For example the third textile area **4** can have a lower mechanical preliminary tension than the first and second textile area. In this manner the third textile area **4** can deform more easily, when tensile stresses occur between the first and the second textile area while the item of clothing **1** is worn. Occurring tensile forces can thus be minimized through an elongation or stretching of the third textile area **4** and the first and second area are isolated.

A low mechanical preliminary tension of the third textile area **4** can be achieved e.g. in that when connecting, e.g. sewing the third textile area **4** to the first and / or second textile area the first and / or second textile area is stretched by means of tensile stress. In this manner, the third textile area **4** receives a lower mechanical preliminary tension than the first and / or second textile area.

The above described embodiments have in common that the provision of a third textile area **4** and its isolating arrangement between the first textile area **2** and the second textile area **3** ensure that the first textile area **2,** which can receive a sensor, moves with the skin of the wearer of the item of clothing **1**. In this manner, a shifting of the sensor is reduced or avoided, i.e. the sensor can continue to perform measurements without interruption. The above described embodiments can for example be combined. Other approaches to isolate the first and second area through a correspondingly implemented third area are also conceivable.

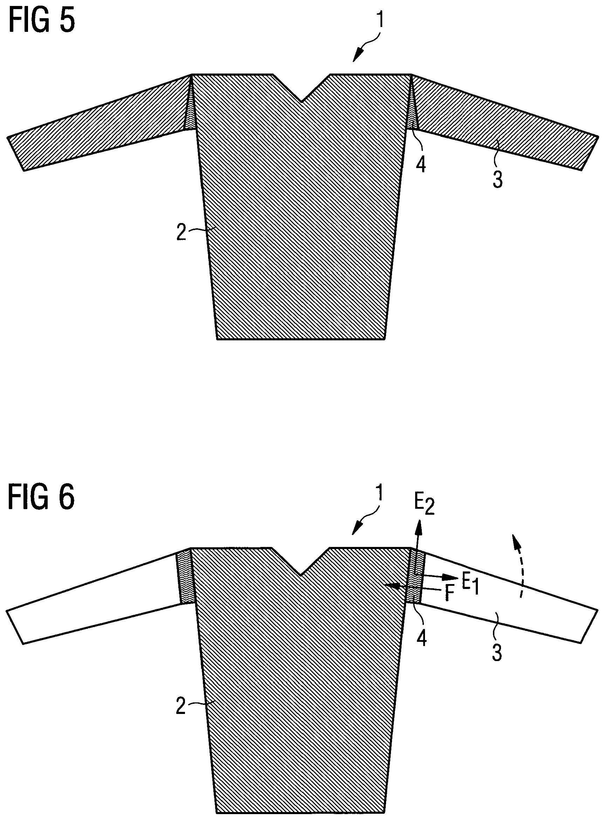

In the embodiments shown in **Figs. 1** to **4** the first textile area **2** does not abut the second textile area **3**, i.e. the third textile area **4** divides these arrears completely. Alternatively, the first and second textile area could be arranged so that they abut, as shown in the embodiment of **Fig. 5**. Here the second textile area **3**, in the form of a sleeve area, is drawn from the top side of the sleeve to the first textile area **2**, in the form of a trunk area, i.e. both areas touch at the top side of the sleeve in the area of the shoulder. The third textile area **4** is arranged on the side and underneath the armpit between the sleeve area **3** and the trunk area **2** in the form of a sleeve portion **4**. Since the greatest tensile forces occur under the armpit between the sleeve area **3** and trunk area **2**, for instance during the raising motion of the arm, this arrangement of the sleeve portion **4** fulfills just as good an isolation function, like in the embodiments of **Figs. 1** to **4**.

In the embodiment of **Fig. 6** the third textile area **4** comprises two different elasticity modules **E₁** and **E₂** in two directions that are different from one another and oriented such that the direction of the smaller elasticity module **E₁** is substantially parallel to the direction in which the greatest mechanical tension **F** occurs when wearing the item of clothing **1.**

Textile materials with different elasticity modules in two directions that are different from one another can be woven fabric, for example. Woven fabrics are made from generally perpendicular warp and weft threads. Varying thicknesses and densities of warp and weft threads and the type of interweaving can cause elasticity modules that distinctly differ from one another in the direction of the warp thread and the direction of the weft thread.

In the embodiment of **Fig. 6** the third textile area **4** is arranged on the item of clothing **1** such that the direction of the smallest elasticity module **E₁** is substantially parallel to the direction in which the greatest mechanical tension **F** occurs when wearing the item of clothing.

In the example of the item of clothing for the upper part of the body of **Fig. 6****,** the greatest mechanical tension **F** occurs at the underside of the armpit, e.g. when lifting the arm up and is directed towards the trunk area **2** from the sleeve area **2**. Correspondingly, the direction of the smallest elasticity module **E₁** is arranged substantially parallel to the direction of this mechanical tension **F.** In this manner a stretching of the third textile area upon occurrence of tensile stress is further favored and a shifting of the sensor is reduced or avoided.

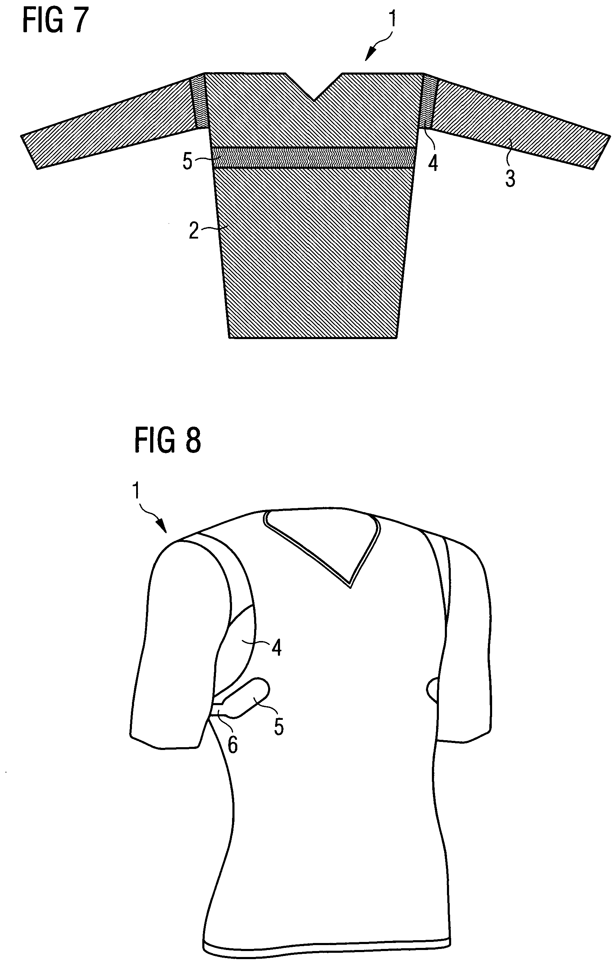

**Fig. 7** shows a further embodiment of the present invention, in which the item of clothing **1** is designed to arrange a sensor **5** circumferentially so that it at least partially runs around the torso of the wearer of the item of clothing **1** when the item of clothing is worn. For example, it can be a meander-shaped electrical conductor, which is used to measure respiration. For measuring respiration the sensor **5**, for example, has meander-shaped electrical conductors which respectively represent an electrical coil and can be connected to an electrical oscillator. Due to the respiratory motions the circumference of the chest and abdomen alter and thus the length of the conductors, the inductance of the coils and finally the oscillation frequency. The alteration of the oscillation frequency can be evaluated and permits conclusions to be made with regard to the respiratory motions.

**Fig. 8** shows a representation of another embodiment of the item of clothing **1** according to the invention, in which the item of clothing **1** comprises a circumferential third textile area **4** and an electrical conductor **6** that is connected to the sensor **5**. The electrical conductor can, for example, lead to a measurement data processing device (not shown in **Fig. 8**), which is arranged at the item of clothing **1** and processes or stores or wirelessly transmits the data measured by sensor **5** to a receiver.

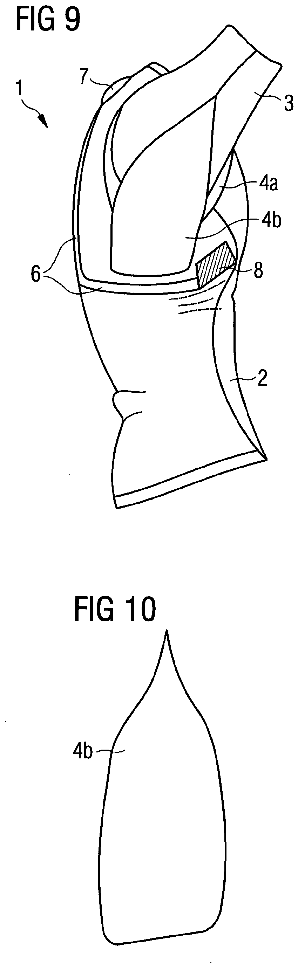

**Fig. 9** shows a further embodiment of the item of clothing **1** according to the invention with a third textile area **4** comprising two sections **4a** and **4b** and a measurement data processing device **7**. The third textile area **4** comprises a section **4a** surrounding the arm in the shoulder area and a longitudinal section **4b** that extends from the lateral thorax area under the armpit to the upper arm area. Furthermore, an area **8** optimal for the function of the sensor is shown in **Fig. 9****.** If the sensor, which in this embodiment is a heart rate electrode, positioned in this area, then it is able to measure the heart rate of a wearer of the item of clothing **1.** The measurement data processing device **7** is connected to the heart rate electrode by means of an electrical conductor **6.** This conductor can be flexible and exhibit a similar stretch behavior to the textile area in which it is arranged.

**Fig. 10** shows a detailed representation of a section **4b** of a third textile area **4** according to the embodiment of **Fig. 9****.** The section **4b** is elongated and has a tapered shape. As a result of this cut, an optimal isolation of the first and second area is made possible.

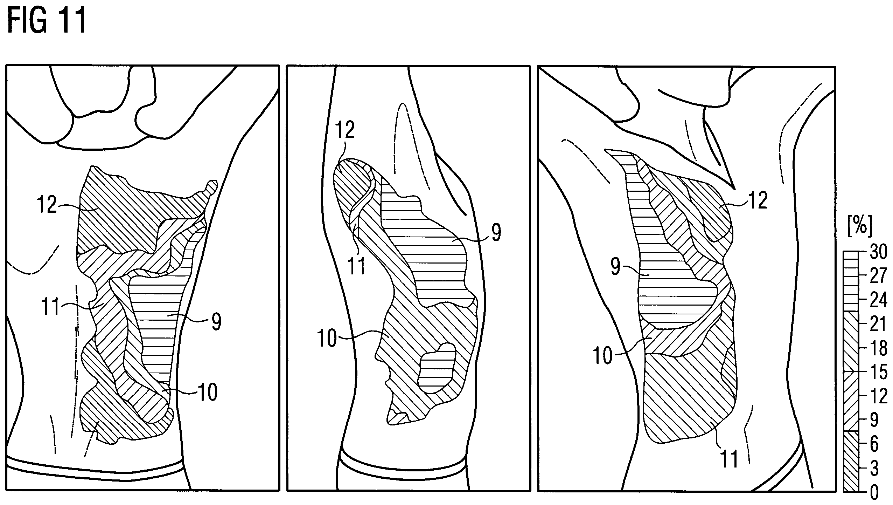

**Fig. 11** shows an illustration of a partial aspect of the performance of an exemplary method. Here, the stretching pattern of the skin of a wearer, namely an athlete, was recorded during a sport-specific motion, namely a throw-in during football, by means of an optical method. In this Figure, the stretching of the skin is shown as a relative stretching compared to the skin's state of rest, for instance during upright casual standing with hanging arms. Thus, a relative stretching of 100% in a specific area of the skin means that the skin in this area has stretched to twice its length. A stretching of 30% would, for example, mean that a previously 1 cm long piece of skin has stretched to a length of 1.3 cm.

As shown in **Fig. 11****,** the greatest stretching of 25% to 60% occurs in area 9. In area 10 a stretching of 20% to 25% is reflected. In area 11, a stretching of 10% to 20% and in area 12, a stretching of 0% to 10% is reflected. As shown in **Fig. 11****,** during a throw-in, the greatest stretching of the skin occurs below the armpit between the mid-lateral thorax area and mid-lateral back area.

After the stretching pattern shown as an example in **Fig. 11** was recorded, an item of sports clothing is manufactured, whereby the item of sports clothing exhibits a substantially similar stretching pattern to the stretching pattern of the skin when worn.

The stretching pattern of the item of sports clothing is similar to the stretching pattern of the skin so much so that a sensor received by the item of sports clothing, such as a heart rate electrode for instance, remains substantially fixed to an area of a subjacent organ of the wearer of the item of sports clothing during the specific motions of a wearer of the item of sports clothing. This means that the sensor does not shift during the specific motions of the wearer such that a measuring is constantly no longer possible.

This exemplary method permits the manufacture of an item of sports clothing, which is suitable to receive a sensor and thus permits a measurement of physiological data of a wearer of the item of sports clothing in real-time during natural motion sequences, without there being a constant "loss" of the measuring signal. For example, in accordance with this method the item of sports clothing can be adapted for a specific type of sport or a group of sports, in that when recording the stretching pattern of the skin at least one motion specific to the sport is performed.
An athlete of a field sport e.g., who wears an item of sports clothing manufactured in accordance with this method, does not need to leave the sports field, in order to correct the position of the sensor. Even in relatively "rough" sports such as rugby, the fixture of the sensor is guaranteed.

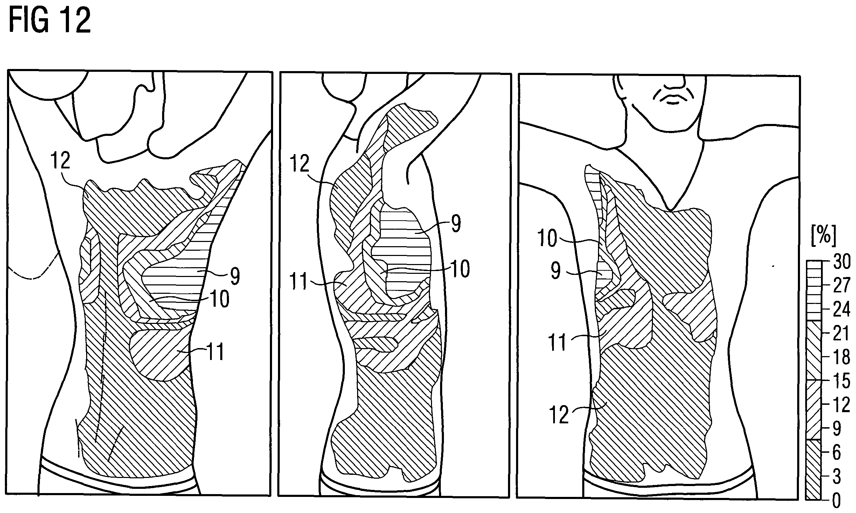

**Fig. 12** shows an illustration of a partial aspect of the performance of the exemplary method. In this Figure, the stretching of an item of sports clothing according **1** to the invention is shown. The stretching of the item of sports clothing was measured here using the same method as the stretching of the skin in Fig. 11. The areas of different stretching correspond to those in Fig. 11. The least stretching, between 0% and 10%, occurs in area 12. This area is best suited for the positioning of the sensor, since the sensor and/or the stretchable electric feed for the sensor, since the sensor and/or the stretchable electric feed will shift the lease or not at all in this area during a sport-specific motion, such as a throw-in for instance. The sensor and/or the stretchable electric feed can also be positioned in area 11, which exhibits a stretching of 10% to 20%. Preferably, a stretchable electric feed for the sensor has the same stretching behavior, e.g. expressed by means of an elasticity module, as the area of the item of sports clothing in which the stretchable electric feed is attached.

The stretching scale, shown in Fig. 11 and 12, ranges from 0% to 30%. However, stretching beyond 30% also occurs in the areas 9 shown in Fig. 11 and 12, for example 60%. In the explanation of Fig. 11 and 12, the stretching was divided into four areas, namely 12: 25% to 60%, 11: 20% to 25%, 10: 10% to 20% and 9: 0% to 10%. Other divisions, grosser or finer, are possible of course. Likewise, the stretching scale can be adapted to larger or smaller maximum occurring stretches, depending on e.g. sport-specific motion or sport.

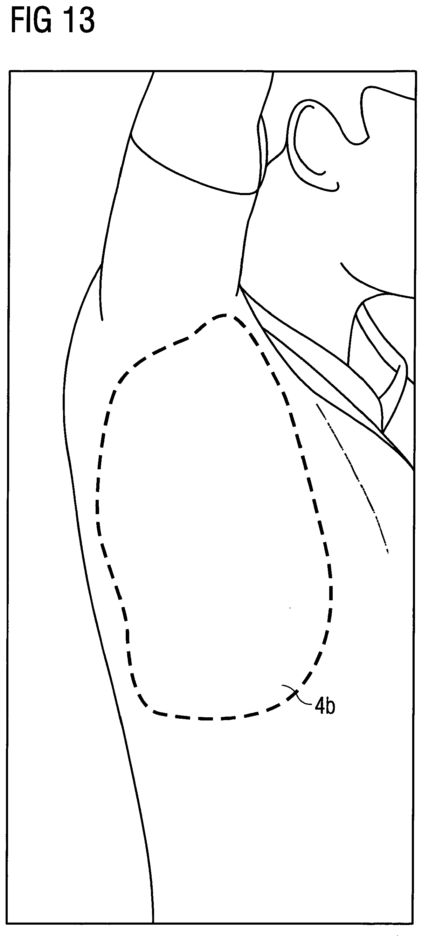

**Fig. 13** shows a detailed representation of a section 4b of a third textile area according to an embodiment. In section 4b, the third textile area has an elasticity of more than 30%, preferably more than 60%.

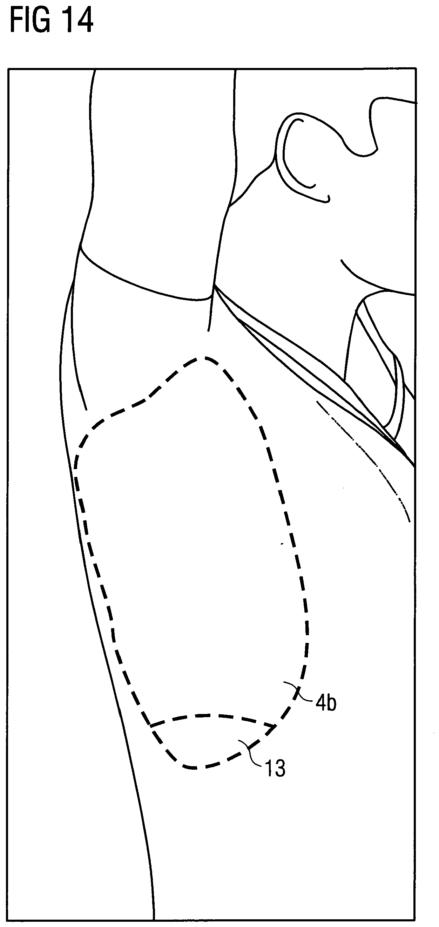

**Fig. 14** shows a detailed representation of a section 4b of a third textile area with a slot 13 according to an embodiment. The slot in the third textile area can additionally favor the isolation between the first and second textile area, in that it prevents or reduces the raising of the first textile area, e.g. during a throw-in during football.

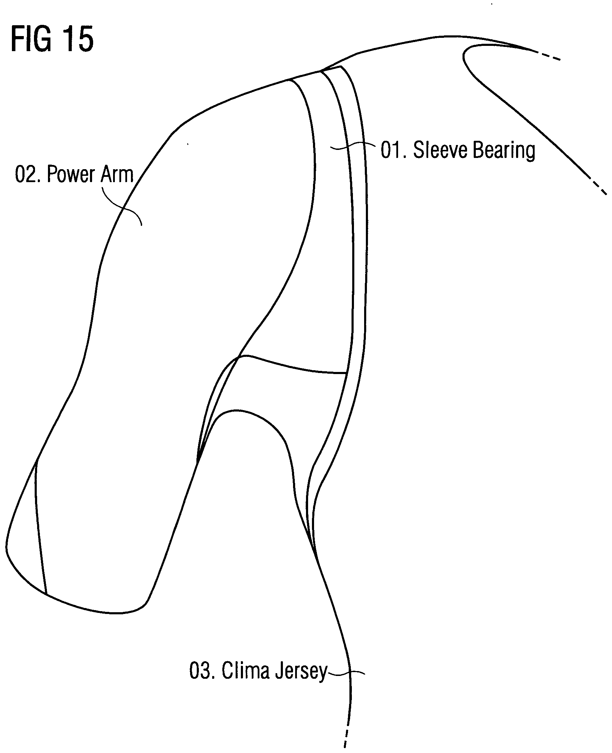

**Fig. 15** shows a partial view of an alternative embodiment of an item of clothing according to the invention. Compared to the previous embodiments, this embodiment has a different cut, particularly in the third area.

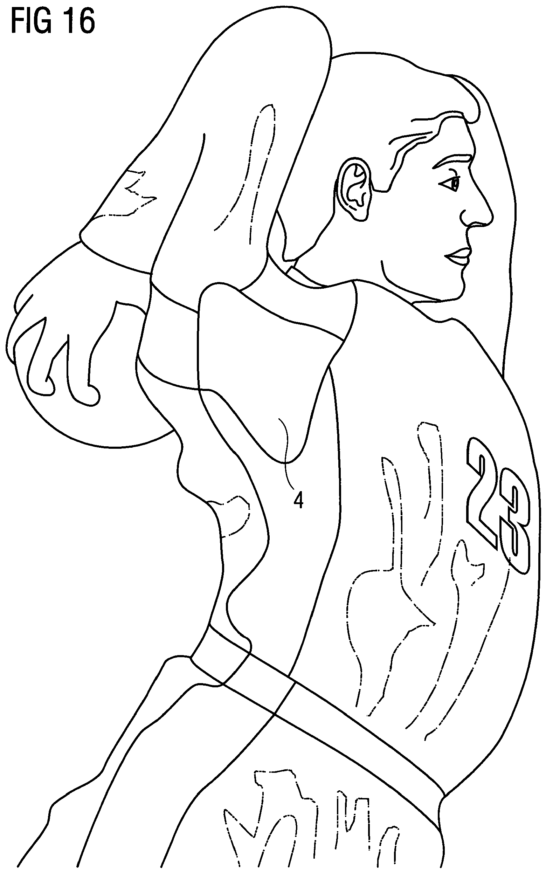

**Fig. 16** shows a partial view of an alternative embodiment of an item of clothing according to the invention, which is suitable for the sport of basketball. Here, a part of the third textile area 4 is positioned below the armpit in the shape of a diamond.

It is important that a sensor received by the item of clothing according to the invention is positioned as optimally as possible. The following method can be applied for this:
The method concerns the determination of an area for possible positions of a sensor on an item of clothing, whereby the sensor is suitable to measure physiological data of a wearer of the item of clothing and whereby the method comprises the steps: a. Putting on the item of clothing by a first wearer; b. Marking areas of possible positions of the sensor on the item of clothing specific to the first wearer; c. Repeating steps a and b for at least a second wearer; and d. Determining an overlapping area of the specific area of possible positions of the sensor of the first and second wearer.

The overlapping area can be determined directly, but also indirectly, for example by means of a reverse conclusion of the examination of all areas, in which the possible sensor positions of the first and second wearer do not overlap. The overlapping area can also be determined in that the area specific for the second wearer is only determined within the specific area for the first wearer. Correspondingly, the area specific for a third wearer would only be determined within the specific area for the second wearer, and so on. In this manner, the overlapping area is continuously narrowed down.

The method ensures that the sensor or a mount therefore is positioned as optimally as possible on the item of clothing, so that a slight shift can be tolerated. Generally, items of clothing are offered in different sizes, for example S (for "small"), M (for "medium"), L (for "large") and XL (for "extra large"), if necessary with increments to smaller and larger sizes. As a rule, items made-to-measure are not particularly found in the leisure and sports sector, but are also possible there - e.g. in high-performance sport. For this reason, one specific size should fit as large a number of different wearer-anatomies as possible. As is know people who share the same cloths size do not share the same anatomy, but rather there are great differences, for instance with regard to the circumference, the arm and leg length, shoulder and hip width, and so on.

The method permits the determination of an area on the item of clothing, which is optimal for the positioning of the sensor for a plurality of different wearer-anatomies. This area is the overlapping area determined by means of the method. As a result of the method it is guaranteed that the sensor or a mount therefore is positioned on the item of clothing such that it lays upon the organ to be measured in a large a number of different wearers as possible in a manner that a measurement of physiological data of said organ is possible. As a result of this positioning a slight shift can be tolerated since the sensor sill lies within the optimal area determined by means of the method. If the sensor were already positioned in an area that was not optimal for the measurement, for instance positioned at a peripheral area of the organ, immediately after the item of clothing is put on, thus prior to any activity, then a slight shift could lead to the sensor distancing itself too far away from the optimal are. A measurement would then no longer be possible.

In a preferred example, the method further comprises the step: Application of a reference mark on the item of clothing. Preferably, this occurs prior to step a. However, it is equally possible not to apply the reference mark until after step a. A reference mark permits the comparison between the specific areas of possible positions of the sensor for different wearers in an easy manner. The electronic measurement and processing in the course of the method are facilitated by means of a reference mark.

Preferably, the reference mark is a grid. This facilitates the measurement of the specific areas of possible positions of the sensor for different wearers, in that the coordinates of the areas can be determined relative to the grid.

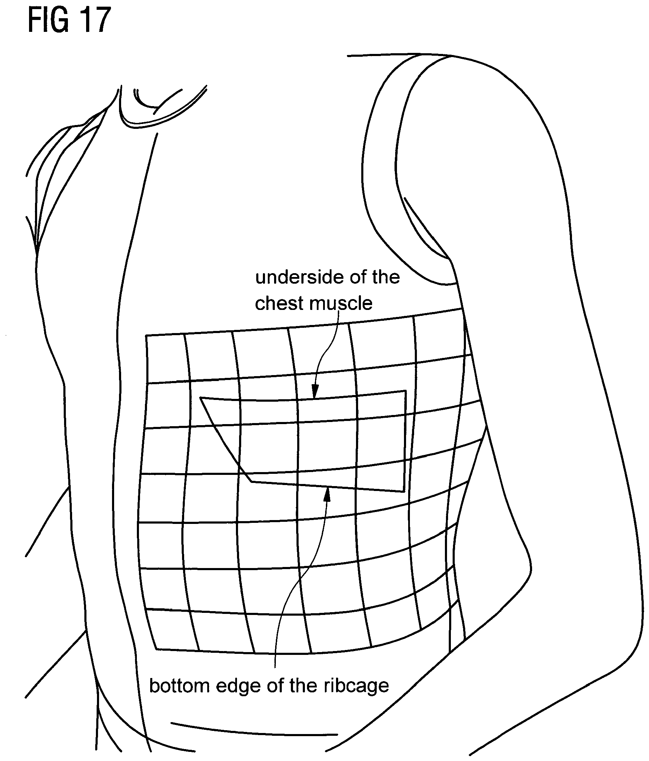

**Fig. 17** shows a representation regarding the finding of an area of optimal position of a sensor. In Fig. 17 a grid is shown as a reference mark. Furthermore, the optimal area for the sensor on the item of clothing was determined for the current wearer and correspondingly marked on the item of clothing. This lies between the underside of the chest muscle (musculus pectoralis major) and the bottom edge of the ribcage, e.g. in the region of the costal arch (arcus costalis). This position permits, on the one hand, a good measuring signal of the sensor in a state of rest of a wearer of the item of clothing. On the other hand, a "loss" of the signal during motions, e.g. during sport, of the wearer are reduced or avoided.

Preferably, the method comprises the step: Electronic recording of the specific areas of possible positions of the senor of the first and second wearers with regard to the reference mark. An electronic recording permits a simple electronic processing and evaluation in the course of the method.

It is further preferred that the method comprises the step: Superimposing the specific areas of possible positions of the sensor of the at least two wearers using the reference mark. Through superimposing the specific areas of possible positions of the sensor of different wearers, the overlapping area can easily be determined as an intersection. However, other methods of direct and indirect determination of the overlapping area are also possible.

In a preferred example of the method, the specific areas of possible positions of the sensor for the first and second wearer are limited by an underside of a chest muscle (musculus pectoralis major) and a bottom edge of the ribcage, e.g. in the region of the costal arch (arcus costalis). Such a limited area is an area that is relevant for the determination of the heartbeat, i.e. a sensor that is suitable for measuring a heartbeat, an electrode for instance, should be positioned in this area.

In a preferred example of the method, the first and second wearers are respectively athletes of different type of sport. It is hereby guaranteed that the position of the sensor is optimal during the performance of different types of sport, such as football and basketball, and that a slight shift of the sensor can be tolerated.

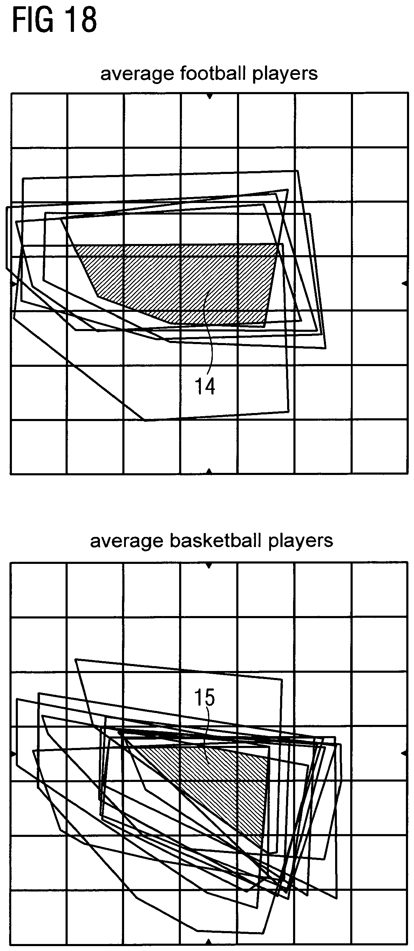

**Fig. 18** shows an illustration of this aspect of the described method for finding an area of optimal position of a sensor. The optimal areas for different average football players is shown on the left hand side, while the optimal areas for different average basketball players is shown on the right hand side. From the different optimal areas of the football players results the overlapping area 14, which is optimal for a large proportion of football players, if the football players used for the performance of the method correspond to the average population and/or buyer. The same applies for the overlapping area 15 on the right hand side for the average basketball players.

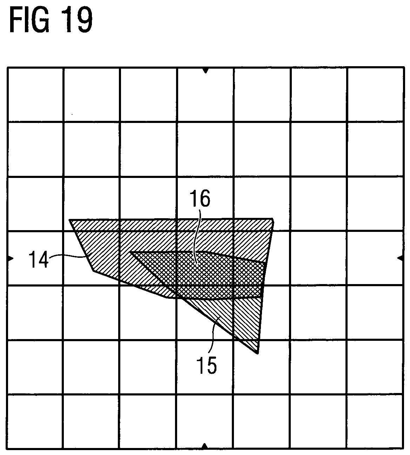

**Fig. 19** shows an illustration of an aspect of the finding of an area of optimal position of a sensor for two different types of sport. In this Figure, the areas 14 and 15 of Fig. 18 have been overlapped so that an area 16 results, in which the sensor is positioned optimally for both football as well as basketball.
In a further preferred example of the method, the specific area for the first or second wearer comprises all positions in which the sensor is able to measure physiological data of the first or second wearer.

A further example concerns an item of clothing with at least one mounting device for a sensor, whereby the position of the mounting device for the sensor in the item of clothing has been determined by means of a method, which comprises the determination of an area of possible positions of the sensor on the item of clothing according to the above described method. The mounting device, i.e. a pocket, fixes the sensor on the item of clothing and thus determines its position on the item of clothing. The position of the sensor and thus the position of the mounting device is determined in accordance with the above described method, so that the position of the sensor and thus that of the mounting device is optimal for a plurality of different wearer-anatomies and so that a slight shift of the sensor can be tolerated, without the measurement performed by means of the sensor is negatively influenced. As a result, the items of clothing differ even from items of clothing for receiving sensors that are previously known from the prior art.

A further example concerns an item of clothing comprising at least one sensor, whereby the position of the sensor in the item of clothing was determined by a method, which comprises the determination of an area of possible positions of the sensor on the item of clothing in accordance with the above described method. It is hereby guaranteed that the position of the sensor is optimal for a plurality of different wearer-anatomies and that a slight shift of the sensor can be tolerated, without the measurement performed by means of the sensor is negatively influenced. It also applies here that the position determination by means of using the exemplary method significantly determines the measurement accuracy and the reliability of the sensor function of the item of clothing according to the invention.

## Claims

1. Item of clothing (1), comprising:
a. a first textile area (2), comprising at least one sensor, whereby the first textile (2) area comprises a pocket for receiving the sensor;
b. a second textile area (3); and
c. a third textile area (4), which is arranged at least partially between the first (2) and second (3) textile areas,
whereby the third textile area (4) is provided such that when the item of clothing (1) is worn it isolates relative motions between the first textile area (2) and the second textile area (3), so that the sensor remains substantially fixed in relation to an area of a subjacent organ of a wearer of the item of clothing (1).

2. Item of clothing (1) according to claim 1, whereby a material processed into the third textile area (4) can be stretched more easily than a material processed into the first textile area (2).

3. Item of clothing (1) according to one of the preceding claims, whereby the third textile area (4) comprises an uneven structure (7).

4. Item of clothing (1) according to one of the preceding claims, whereby the third textile area (4) is provided such that when the item of clothing (1) is worn it is spaced further away from the skin of a wearer of the item of clothing (1) than the first (2) textile area.

5. Item of clothing (1) according to one of the preceding claims, whereby the third textile area (4) has a substantially different mechanical preliminary tension than the first (2) and / or the second (3) textile area.

6. Item of clothing (1) according to one of the preceding claims, whereby the third textile area (4) comprises a longish section substantially along the direction of greatest stretching when wearing the item of clothing.

7. Item of clothing (1) according to one of the preceding claims, whereby the organ is the skin, the heart, the lung, the thorax or the abdomen of a wearer of the item of clothing.

8. Item of clothing (1) according to one of the preceding claims, whereby the third textile area (4) is arranged between the first (2) and second (3) textile areas such that the first (2) and second (3) textile areas do not abut.

9. Item of clothing (1) according to one of the preceding claims, whereby it is an item of clothing for the upper part of the body, whereby the first textile area (2) is a trunk area and whereby the second textile area (3) is sleeve area.

10. Item of clothing (1) according to claim 9, whereby the third textile area (4) is a circumferential sleeve piece.

11. Item of clothing (1) according to one of the preceding claims, whereby the third textile area is a fabric.

12. Item of clothing (1) according to one of the preceding claims, whereby the first and second textile areas have the same material.

13. Item of clothing (1) according to one of the preceding claims, whereby the third textile area comprises two different elasticity modules in two directions that are different from one another and oriented such that the direction of the smaller elasticity module is substantially parallel to the direction in which the greatest mechanical tension occurs when wearing the item of clothing.

14. Item of clothing (1) according to one of the preceding claims, whereby the item of clothing (1) is designed to arrange the sensor such that it is positioned on one side of the torso of a wearer of the item of clothing (1) when the item of clothing (1) is worn.

## Patentansprüche

1. Kleidungsstück (1) umfassend:
a. ein erster Textilbereich (2), umfassend zumindest einen Sensor, wobei der erste Textilbereich (2) eine Tasche zum Empfangen des Sensors umfasst;
b. ein zweiter Textilbereich (3); und
c. ein dritter Textilbereich (4), welcher zumindest teilweise zwischen dem ersten (2) und zweiten (3) Textilbereich angeordnet ist,
wobei der dritte Textilbereich (4) so bereitgestellt ist, dass wenn ein Kleidungsstück (1) getragen wird, es relative Bewegungen zwischen dem ersten Textilbereich (2) und dem zweiten Textilbereich (3) isoliert, so dass der Sensor im Wesentlichen fixiert bleibt in Bezug auf ein Gebiet eines benachbarten Organs eines Trägers des Kleidungsstücks (1).

2. Kleidungsstück (1) gemäß Anspruch 1, wobei ein Material, welches in den dritten Textilbereich (4) eingearbeitet ist, einfacher gedehnt werden kann, als ein Material, welches in den ersten Textilbereich (2) eingearbeitet ist.

3. Kleidungsstücke (1) gemäß einem der vorhergehenden Ansprüche, wobei der dritte Textilbereich (4) eine unebene Struktur (7) umfasst.

4. Kleidungsstück (1) gemäß einem der vorhergehenden Ansprüche, wobei der dritte Textilbereich (4) so bereitgestellt ist, dass, wenn das Kleidungsstück (1) getragen wird, es weiter von der Haut eines Trägers des Kleidungsstücks (1) beabstandet ist, als der erste (2) Textilbereich.

5. Kleidungsstück (1) gemäß einem der vorhergehenden Ansprüche, wobei der dritte Textilbereich (4) eine im Wesentlichen unterschiedliche mechanische Vorspannung als der erste (2) und/oder der zweite (3) Textilbereich aufweist.

6. Kleidungsstück (1) gemäß einem der vorhergehenden Ansprüche, wobei der dritte Textilbereich (4) einen länglichen Abschnitt, im Wesentlichen entlang der Richtung der größten Dehnung beim Tragen des Kleidungsstücks, umfasst.

7. Kleidungsstück (1) gemäß einem der vorhergehenden Ansprüche, wobei das Organ die Haut, das Herz, die Lunge, der Thorax oder das Abdomen eines Trägers des Kleidungsstücks ist.

8. Kleidungsstück (1) gemäß einem der vorhergehenden Ansprüche, wobei der dritte Textilbereich (4) zwischen dem ersten (2) und zweiten (3) Textilbereich angeordnet ist, so dass sich der erste (2) und zweite (3) Textilbereich nicht berühren.

9. Kleidungsstück (1) gemäß einem der vorhergehenden Ansprüche, wobei es ein Kleidungsstück für den oberen Teil des Körpers ist, wobei der erste Textilbereich (2) ein Rumpfbereich ist und wobei der zweite Textilbereich (3) ein Ärmelbereich ist.

10. Kleidungsstück (1) gemäß Anspruch 9, wobei der dritte Textilbereich (4) ein umfängliches Ärmelstück ist.

11. Kleidungsstück (1) gemäß einem der vorhergehenden Ansprüche, wobei der dritte Textilbereich ein Stoff ist.

12. Kleidungsstück (1) gemäß einem der vorhergehenden Ansprüche, wobei der erste und zweite Textilbereich dasselbe Material aufweisen.

13. Kleidungsstück (1) gemäß einem der vorhergehenden Ansprüche, wobei der dritte Textilbereich zwei unterschiedliche Elastizitätsmodule in zwei Richtungen, welche voneinander verschieden sind, umfasst, welche so ausgerichtet sind, dass die Richtung des kleineren Elastizitätsmoduls im Wesentlichen parallel zur Richtung ist, in welcher die größte mechanische Spannung auftritt beim Tragen des Kleidungsstücks.

14. Kleidungsstück (1) gemäß einem der vorhergehenden Ansprüche, wobei das Kleidungsstück (1) konzipiert ist, um den Sensor so anzuordnen, dass er an einer Seite des Torsos eines Trägers des Kleidungsstücks (1) angeordnet ist, wenn das Kleidungsstück (1) getragen wird.

## Revendications

1. Article vestimentaire (1), comprenant :
a. une première zone textile (2), comprenant au moins un capteur, la première zone textile (2) comprenant une poche pour recevoir le capteur ;
b. une seconde zone textile (3) ; et
c. une troisième zone textile (4), qui est au moins partiellement disposée entre la première (2) et la seconde (3) zone textile,
la troisième zone textile (4) étant agencée de telle sorte que lorsque l'article vestimentaire (1) est porté il isole les déplacements relatifs entre la première zone textile (2) et la seconde zone textile (3), afin que le capteur reste substantiellement fixe par rapport à une zone d'un organe sous-jacent d'un porteur de l'article vestimentaire (1).

2. Article vestimentaire (1) selon la revendication 1, dans lequel un matériau traité dans la troisième zone textile (4) peut être étiré plus facilement qu'un matériau traité dans la première zone textile (2).

3. Article vestimentaire (1) selon l'une des revendications précédentes, dans lequel la troisième zone textile (4) comprend une structure non uniforme (7).

4. Article vestimentaire (1) selon l'une des revendications précédentes, dans lequel la troisième zone textile (4) est agencée de telle sorte que lorsque l'article vestimentaire (1) est porté elle est plus éloignée de la peau d'un porteur de l'article vestimentaire (1) que la première zone textile (2).

5. Article vestimentaire (1) selon l'une des revendications précédentes, dans lequel la troisième zone textile présente une tension préliminaire mécanique substantiellement différente de celle de la première (2) et/ou de la seconde (3) zone textile.

6. Article vestimentaire (1) selon l'une des revendications précédentes, dans lequel la troisième zone textile (4) comprend une section oblongue substantiellement le long de la direction de plus grand étirement lorsque l'article vestimentaire est porté.

7. Article vestimentaire (1) selon l'une des revendications précédentes, dans lequel l'organe est la peau, le coeur, le poumon, le thorax ou l'abdomen d'un porteur de l'article vestimentaire.

8. Article vestimentaire (1) selon l'une des revendications précédentes, dans lequel la troisième zone textile (4) est agencée entre la première (2) et la seconde (3) zone textile de telle sorte que la première (2) et la seconde (3) zone textile ne se touchent pas.

9. Article vestimentaire (1) selon l'une des revendications précédentes, dans lequel il s'agit d'un article vestimentaire pour le haut du corps, la première zone textile (2) étant une zone pour le torse et la seconde zone textile (3) étant une zone de manche.

10. Article vestimentaire (1) selon la revendication 9, dans lequel la troisième zone textile (4) est une pièce de manche circonférentielle.

11. Article vestimentaire (1) selon l'une des revendications précédentes, dans lequel la troisième zone textile est un tissu.

12. Article vestimentaire (1) selon l'une des revendications précédentes, dans lequel la première et la seconde zone textile sont de la même matière.

13. Article vestimentaire (1) selon l'une des revendications précédentes, dans lequel la troisième zone textile présente deux modules d'élasticité différents dans deux directions qui sont différentes l'une de l'autre et sont orientées de telle sorte que la direction de moindre module d'élasticité soit substantiellement parallèle à la direction dans laquelle est subie la plus grande tension mécanique lorsque l'article vestimentaire est porté.

14. Article vestimentaire (1) selon l'une des revendications précédentes, dans lequel l'article vestimentaire (1) est conçu pour disposer le capteur de telle sorte qu'il soit positionné d'un côté du torse d'un porteur de l'article vestimentaire (1) lorsque l'article vestimentaire (1) est porté.
